# EUROPÄISCHE PATENTANMELDUNG

(11) **EP 1 671 970 A1**
(43) Veröffentlichungstag der Anmeldung: **21.06.2006**
(21) Anmeldenummer: 05026671.7
(22) Anmeldetag: 07.12.2005
(51) Int. Cl.: C07D 471/04

(54) **Substituierte 1H-Pyrrolo(2,3-b)pyridine und deren Herstellung**

(30) Priorität: 15.12.2004 DE 102004060659
(71) Anmelder: LANXESS Deutschland GmbH, 51369 Leverkusen (DE)
(72) Erfinder: Ebenbeck, Wolfgang Dr., 51373 Leverkusen (DE); Santiago Figueroa, Perez Dr., 51373 Leverkusen (DE); Schirok, Hartmut Dr., 42287 Wuppertal (DE)

(57) **Zusammenfassung**

Die Erfindung betrifft substituierte 1*H*-Pyrrolo[2,3-b]pyridine der allgemeinen Formel (I),

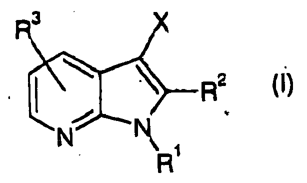

Verfahren zu deren Herstellung sowie Zwischenprodukte hierzu.

## Beschreibung

Die Erfindung betrifft neue substituierte 1*H*-Pyrrolo[2,3-b]pyridine, Verfahren zu deren Herstellung sowie Zwischenprodukte hierzu.

1*H*-Pyrrolo[2,3-b]pyridine sind wichtige Zwischenprodukte für die Herstellung pharmazeutischer Wirkstoffe (vgl. WO-A 2004/009601 oder WO-A 2004/039796). Außerdem können substituierte 1*H*-Pyrrolo[2,3-b]pyridine sowie verwandte Verbindungen selbst pharmazeutische Wirksamkeit aufweisen und als Wirkstoffe in pharmazeutischen Zusammensetzungen eingesetzt werden (vgl. WO-A 03/082289, WO-A 2004/016609 und WO-A 2004/009601). So beschriebt beispielsweise WO-A 2004/016609 die Verwendung von substituierten 1*H*-Pyrrolo[2,3-b]pyridinen als Inhibitoren der Kinase Itk, WO-A 2004/009601 die Verwendung von Wirkstoffen, die ein 1*H-*Pyrrolo[2,3-b]pyridin-Fragment enthalten, als Tyrosinkinase-Aktivitätshemmer von Wachstumsrezeptoren, weshalb diese für den Einsatz als Anti-Krebsmittel geeignet sind, und WO-A 03/082289 beschreibt die Möglichkeit, substituierte 1*H*-Pyrrolo[2,3-b]pyridine aufgrund ihrer antiviralen Wirkung für die Behandlung von HIV und AIDS einzusetzen.

Aufgrund des vielfältigen Wirkungspotential von 1*H*-Pyrrolo[2,3-b]pyridinen oder Verbindungen enthaltend 1*H*-Pyrrolo[2,3-b]pyridin-Bausteine, bestand weiterhin Bedarf an weiteren solcher substituierten 1*H*-Pyrrolo[2,3-b]pyridine, die beispielsweise für die Herstellung pharmazeutischer Wirkstoffe oder als Wirkstoffe selbst für die Behandlung anderer Krankheiten geeignet oder den bekannten Verbindungen beispielsweise hinsichtlich Löslichkeit, Wirkhöhe oder Pharmakokinetik überlegen sind.

Somit bestand die Aufgabe der vorliegenden Erfindung darin, solche neuen substituierten 1*H-*Pyrrolo[2,3-b]pyridine aufzufinden.

Diese Aufgabe wurde durch die Bereitstellung der erfindungsgemäßen Verbindungen der allgemeinen Formel (I) gelöst. Keine dieser Verbindungen ist bisher im Stand der Technik beschrieben.

Gegenstand der vorliegenden Erfindung sind somit Verbindungen der allgemeinen Formel (I), 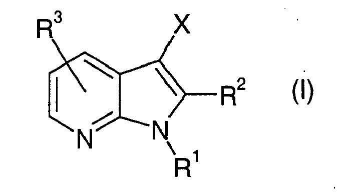
- R¹: für H oder gegebenenfalls substituiertes lineares, verzweigtes oder cylisches C₁-C₁₈-Alkyl, bevorzugt C₁-C₆-Alkyl sowie C₃-C₆-Cycloalkyl, C₁-C₁₈-Alkoxy, bevorzugt C₁-C₆-Alkoxy, C₂-C₆-Alkenyl, C₄-C₆-Cycloalkenyl, C₂-C₆-Alkinyl, C₄-C₂₄-Aryl, bevorzugt C₆-C₂₄-Aryl, C₅-C₁₈-Arylalkyl, C₅-C₁₈-Aryloxy, C₅-C₁₈-Arylalkoxy, -SO₂R^{x}, -SO₂NR^{x}R^{y}, -C(O)OR^{x} oder-C(O)NR^{x}R^{y}, Tri-C₁-C₁₈-alkylsilyl-Gruppen sowie weitere geeignete NH-Schutzgruppen für aromatische Verbindungen,
worin R^{x} und R^{y} unabhängig voneinander für H oder gegebenenfalls substituiertes lineares, verzweigtes oder cylisches C₁-C₁₈-Alkyl, bevorzugt C₁-C₆-Alkyl sowie C₃-C₆-Cycloalkyl, C₁-C₁₈-Alkoxy, bevorzugt C₁-C₆-Alkoxy, C₂-C₆-Alkenyl, C₄-C₆-Cycloalkenyl, C₂-C₆₋Alkinyl, C₄-C₂₄-Aryl, bevorzugt C₆-C₂₄-Aryl, C₅-C₁₈-Arylalkyl, C₅-C₁₈-Aryloxy oder C₅₋C₁₈-Arylalkoxy steht
- R², R³: unabhängig voneinander für H, Cl, F, Br, NO₂ Pseudohalogen, Formyl oder geschütztes Formyl, Carboxyl, C(S)NH₂, C(O)NH₂, gegebenenfalls substituiertes lineares, verzweigtes oder cylisches C₁-C₁₈-Alkyl, bevorzugt C₁-C₆-Alkyl sowie C₃-C₆-Cycloalkyl, C₁-C₁₈₋Fluoralkyl, bevorzugt C₁-C₆-Fluoralkyl, C₁-C₁₈-Alkoxy, bevorzugt C₁-C₆-Alkoxy, C₁-C₁₈₋Fluoralkoxy, bevorzugt C₁-C₆-Fluoralkoxy, C₂-C₆-Alkenyl, C₄-C₆-Cycloalkenyl, C₂-C₆₋Alkinyl, C₄-C₂₄-Aryl, bevorzugt C₆-C₂₄-Aryl, C₅-C₁₈-Arylalkyl oder C₅-C₁₈-Arylalkoxy steht, wobei für den Fall, dass R³ für C₅-C₁₈-Arylalkyl steht und dieser über den Alkylteil des C₅₋C₁₈-Arylalkylrestes an den Pyridinring gebunden ist, dieser Alkylteil wenigstens zwei Kohlenstoffatome zwischen Arylteil des C₅-C₁₈-Arylalkylrestes und Pyridinring aufweist, und
- X: für C₁-C₁₈-Fluoralkyl, bevorzugt C₁-C₆-Fluoralkyl, steht.

Ebenfalls Gegenstand der vorliegenden Erfindung sind Salze der Verbindungen der allgemeinen Formel (I).

Weitere geeignete NH-Schutzgruppen in aromatischen Verbindungen, die für R¹ in Frage kommen sind dem Fachmann beispielsweise aus Greene T. W., Wuts P. G. W. in Protective Groups in Organic Synthesis; John Wiley & Sons, Inc. 1999, Protection for Imidazoles, Pyrazoles, Indoles, and other aromatic heterocycles, S.615-631 bekannt.

Als Beispiele für die oben aufgeführten für R¹ geeigneten Reste seien die folgenden genannt: - C(O)OR^{x} oder -C(O)NR^{x}R^{y} können im Rahmen der Erfindung beispielsweise Carbamate bzw. Oxycarbonyl-Gruppen, wie z.B. gegebenenfalls substiuierte C₁-C₁₈-Alkoxycarbonyl-, Tri-C₁-C₁₈₋alkylsiloxycarbonyl, C₃-C₆-Cycloalkoxycarbonylgruppen, insbesondere 2,2,2-Trichlorethoxycarbonyl-, 2-(Trimethylsilyl)-ethoxycarbonyl-, tert.Butoxycarbonyl-, 2,4-Dimethylpent-3-yl-oxycarbonyl-, Cyclohexyl-oxycarbonyl-, 1,1-Dimethyl-2,2,2-trichlorethoxycarbonyl-, Adamantyloxycarbonyl-Gruppen sein, -SO₂R^{x} oder -SO₂NR^{x}R^{y} können beispielsweise gegebenenfalls substituierte N,N-Di-C₁-C₁₈-alkylsulfonyl-, C₄-C₂₄-Arylsulfonyl-Gruppen, insbesondere o-, m-, p-Toluolsulfonyl-, Phenylsulfonyl-, p-Methoxyphenylsulfonyl-, Mesitylensulfonyl-, N,N-Dimethylsulfonyl-Gruppen sein, gegebenenfalls substituierte C₁-C₁₈₋Alkyl- oder C₄-C₂₄-Aryl-Gruppen können beispielsweise Vinyl-, 2-Chlorethyl-, 1-Ethoxyethyl-, 2-(2'-Pyridyl)ethyl-, 2-(4-Nitrophenyl)ethyl-Gruppen und Allyl-, Benzyl-, p-Methoxybenzyl-, 3,4-Dimethoxybenzyl-, 3-Methoxy- bzw. 3,5-Dimethoxybenzyl-, 2-Nitrobenzyl-, 2,4-Dinitrophenyl-, Phenacyl-, Triphenylmethyl-Gruppen, gegebenenfalls substituierte Tri-C₁-C₁₈-alkylsilyl-Gruppen können beispielsweise t.Butyldimethylsilyl-, Triisopropylsilyl-Gruppen, gegebenenfalls substituierte C₁-C₁₈-Alkylgrupen können beispielsweise auch Dimethylaminoethyl-Gruppen, gegebenenfalls substituierte C₃-C₆-Cycloalkylgruppen können beispielsweise 2-Tetrahydropyranyl-Gruppen, gegebenenfalls substituierte C₁-C₁₈-Alkoxygruppen können beispielsweise Methoxymethyl-, Diethoxymethyl-, 2-Chlorethoxymethyl-, 2-(Trimethylsilyl)ethoxymethyl-, t-Butoxymethyl-, t-Butyldimethylsiloxymethyl-, Pivaloyloxymethyl-Gruppen, gegebenenfalls substituierte C₃-C₆-Cycloalkoxy-, Benzyloxymethyl-Gruppen und gegebenenfalls substituierte C₁₋C₁₈-Alkyl-, C₄-C₂₄-Aryl-oder C₅-C₁₈-Arylalkyl-Amide können beispielsweise Hydroxycarbonyl-, Formyl-, N,N-Diethylaminocarbonyl-, Dichlormethylcarbonyl-, t-Butylcarbonyl-, Diphenylthiophosphin-Gruppen sein. Die vorangehende Aufstellung dient der beispielhaften Erläuterung und ist nicht als abschließend zu betrachten.

Bevorzugt Gegenstand der vorliegenden Erfindung sind Verbindungen der allgemeinen Formel (I), worin R¹ für H, -SO₂R^{x}, -SO₂NR^{x}R^{y}, -C(O)OR^{x} oder -C(O)NR^{x}R^{y} steht, worin R^{x} und R^{y} unabhängig voneinander für H oder gegebenenfalls substituiertes C₁-C₁₈-Alkyl, C₄-C₂₄-Aryl oder C₅-C₁₈-Arylalkyl stehen, R¹ bevorzugt für H, o-, m-, p-Toluolsulfonyl oder t-Butyloxycarbonyl steht. In bevorzugten Ausführungsformen steht R¹ für -SO₂R^{x} oder -C(O)OR^{x}, worin R^{x} für gegebenenfalls substituiertes C₁-C₁₈-Alkyl, C₄-C₂₄-Aryl oder C₅-C₁₈-Arylalkyl steht, bevorzugt für p-Toluolsulfonyl oder t-Butyloxycarbonyl.

Bevorzugt Gegenstand der vorliegenden Erfindung sind weiterhin Verbindungen der allgemeinen Formel (I), worin R² für H, C₁-C₆-Alkyl, C₆-C₂₄-Aryl oder C₅-C₁₈-Arylalkyl steht.

Bevorzugt Gegenstand der vorliegenden Erfindung sind weiterhin Verbindungen der allgemeinen Formel (I), worin R³ für H, Cl, F, Br, NO₂, CN, Formyl oder geschütztes Formyl, C₁-C₆-Alkyl, C₁₋C₆-Fluoralkyl, C₁-C₆-Alkoxy, C₁-C₆-Fluoralkoxy, C₆-C₂₄-Aryl, C₅-C₁₈-Arylalkyl, C₅-C₁₈₋Arylalkoxy, bevorzugt für H oder C1 steht.

Bevorzugt Gegenstand der vorliegenden Erfindung sind weiterhin Verbindungen der allgemeinen Formel (I), worin X für C₁-C₆-Fluoralkyl, bevorzugt für CF₃, CF₂CF₃ oder CF(CF₃)₂, besonders bevorzugt für CF₃ steht.

Auch Mischungen enthaltend eine oder mehrere der vorangehend genannten Verbindung(en) der allgemeinen Formel (I) sind Gegenstand der vorliegenden Erfindung.

Die Verbindungen der allgemeinen Formel (I) können je nach Bedeutung von R¹, R² oder R³ in enantiomeren oder diastereomeren Formen auftreten. Von der Erfindung sind alle Enantiomeren, Diastereomeren, Racemate, Mischungen der Enantiomeren oder Diastereomeren in beliebigen Molverhältnissen umfasst.

Des Weiteren können die Verbindungen der allgemeinen Formel (I) je nach Bedeutung von R¹, R² oder R³ in verschiedenen tautomeren Formen auftreten. Von der Erfindung sind ebenfalls alle möglichen tautomeren Formen sowie Mischungen aus diesen in beliebigen Molverhältnissen umfasst.

Salze der erfindungsgemäßen Verbindungen können physiologisch unbedenkliche Salze der erfmdungsgemäßen Verbindungen der allgemeinen Formel (I) mit anorganischen oder organischen Säuren sein. Bevorzugt werden Salze mit anorganischen Säure wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Trifluoressigsäure, Propionsäure, Maleinsäure, Fumarsäure, Äpfelsäure, Zitronensäure, Weinsäure, Milchsäure, Benzoesäure oder Methansulfonsäure, Ethansulfonsäure, Benzolsulfonsäure, Toluolsulfonsäure oder Naphthalinsulfonsäure.

Salze der erfindungsgemäßen Verbindungen können je nach Bedeutung von R¹, R² oder R³ ebenso physiologisch unbedenkliche Salze der erfindungsgemäßen Verbindungen der allgemeinen Formel (I) mit üblichen Basen sein, wie beispielsweise Alkalimetallsalze, wie z.B. Natrium- oder Kaliumsalze, Erdalkalimetallsalze, wie z.B. Calcium- oder Magnesiumsalze, Ammoniumsalze, abgeleitet von Ammoniak oder organischen Aminen, wie beispielsweise Diethylamin, Triethylamin, Ethyldiispropylamin, Prokain, Dibenzylamin, N-Methylmorpholin, Dihydroabietylamin oder Methylpiperidin, oder Salze von Ethanolaminen, wie beispielsweise 2-Diethylaminoethanol, 2-[(2-Hydroxy-ethyl)methyl-amino]-ethanol.

**Alkyl, Alkenyl, Alkinyl** oder **Alkoxy** bedeutet jeweils unabhängig einen linearen, cyclischen, verzweigten oder unverzweigten Alkyl-, Alkenyl-, Alkinyl- oder Alkoxy-Rest. Gleiches gilt für den nichtaromatischen Teil eines Arylalkyl-Restes sowie für Alkyl-, Alkylen- oder Alkoxybestandteile komplexerer Substituenten wie z.B. Alkyl-Sulfonyl, Alkyl-Oxycarbonyl etc..

**C**_{**1**}**-C**_{**6**}**-Alkyl** steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C**_{**1**}**-C**_{**18**}**-Alkyl** darüber hinaus beispielsweise für n-Heptyl und n-Octyl, Pinakolyl, Adamantyl, die isomeren Menthyle, n-Nonyl, n-Decyl, n-Dodecyl, n-Tridecyl, n-Tetradecyl, n-Hexadecyl oder n-Octadecyl.

**C**_{**3**}**-C**_{**6**}**-Cycloalkyl** steht beispielsweise für Cyclopropyl, Cyclobutyl, Cyclopentyl oder Cyclohexyl.

**C**_{**1**}**-C**_{**6**}**-Alkoxy** steht beispielsweise für die den vorangehenden Alkylgruppen entsprechenden Alkoxygruppen, wie z.B. Methoxy, Ethoxy, n-Propoxy, iso-Propoxy, n-Butoxy, sec.-Butoxy, tert.-Butoxy etc.. Auch **C**_{**1**}**-C**_{**18**}**-Alkoxy** steht beispielsweise für die den vorangehenden Alkylgruppen entsprechenden Alkoxygruppen.

**C**_{**1**}**-C**_{**6**}**-Fluoralkyl** bzw. **C**_{**1**}**-C**_{**18**}**-Fluoralkyl** steht beispielsweise für die den vorangehenden Alkylgruppen entsprechenden teilfluorierten oder perfluorierten Alkylgruppen. **C**_{**1**}**-C**_{**6**}**-Fluoralkoxy** bzw. **C**_{**1**}**-C**_{**18**}**-Fluoralkoxy** steht beispielsweise für die den vorangehenden Alkoxygruppen entsprechenden teilfluorierten oder perfluorierten Alkoxygruppen.

**C**_{**2**}**-C**_{**6**}**-Alkenyl** steht beispielsweise für die den vorangehenden Alkylgruppen entsprechenden Alkenylgruppen, wie z.B. Ethenyl, Propenyl, Butenyl, Pentenyl oder Hexenyl. **C**_{**2**}**-C**_{**6**}**-Alkinyl** steht beispielsweise für die den vorangehenden Alkylgruppen entsprechenden Alkinylgruppen, wie z.B. Ethinyl, Propinyl, Butinyl, Pentinyl oder Hexinyl. **C**_{**4**}**-C**_{**6**}**-Cycloalkenyl** steht beispielsweise für Cyclobutenyl, Cyclopentenyl oder Cyclohexenyl.

**Aryl** steht jeweils unabhängig für einen aromatischen Rest mit 4 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 24 Gerüstkohlenstoffatomen. Gleiches gilt für den aromatischen Teil eines Arylalkyl-Restes sowie für Arylbestandteile komplexerer Substituenten wie z.B. Aryl-Sulfonyl, Aryl-Oxycarbonyl.

Beispiele für **C**_{**6**}**-C**_{**24**}**-Aryl** sind Phenyl, o-, p-, m-Tolyl, Naphthyl, Phenanthrenyl, Anthracenyl oder Fluorenyl, Beispiele für heteroaromatisches **C**_{**4**}**-C**_{**24**}**-Aryl** in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind Pyridyl, Pyridyl-N-Oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl, Benzofuranyl oder Dibenzofuranyl.

**Arylalkyl** bedeutet jeweils unabhängig einen geradkettigen, cyclischem, verzweigten oder unverzweigten Alkyl-Rest nach obiger Definition, der einfach, mehrfach oder vollständig durch Aryl-Reste gemäß obiger Definition substituiert sein kann.

**C**_{**5**}**-C**_{**18**}**-Arylalkyl** steht beispielsweise für Benzyl oder (R)- oder (S)-1-Phenylethyl:

**Halogen** kann für Fluor, Chlor, Brom oder Iod stehen. **Pseudohalogen** kann beispielsweise für Cyanid, Cyanat oder Thiocyanat stehen.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Als mögliche Substituenten für die Reste R¹ bis R³ kommen zahlreiche organische Gruppen in Frage, beispielsweise Alkyl-, Cycloalkyl-, Aryl-, Halogen, Hydroxyl-, Ether-, Thioether-, Disulfid-, Sulfoxid-, Sulfonsäure-, Sulfonat-, geschützte Amino-, Aldehyd-, Keto-, Carbonsäureester-, Carbonat-, Carboxylat-, Cyano-, Alkylsilan- und Alkoxysilangruppen sowie gegebenenfalls geschützten Carboxylamidgruppen. Für den Fall, dass R³ für einen arylsubstituierten C₁-C₁₈₋Alkylrest steht, muss dieser Alkylrest wenigstens zwei Kohlenstoffatome zwischen Arylsubstituenten und Pyridinring aufweisen.

Beispielhaft für Verbindungen der allgemeinen Formel (I) seien die folgenden Verbindungen der Formeln (I-1) bis (I-4) aufgeführt:

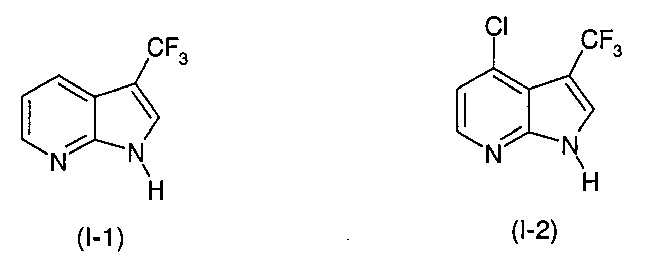

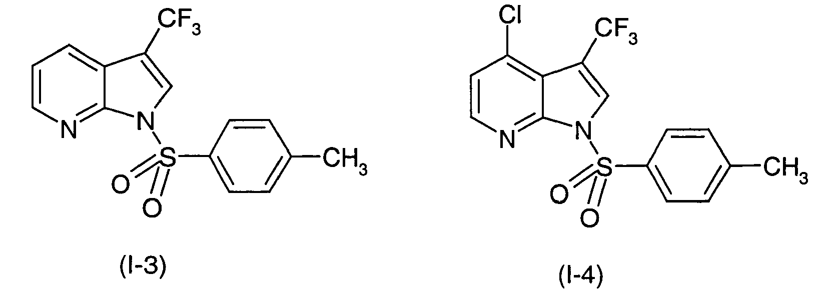

Die erfindungsgemäßen Verbindungen der allgemeinen Formel (I) lassen sich auf verschiedenen Wegen auf einfache Weise herstellen.

Beispielsweise können die Verbindungen der allgemeinen Formel (I) aus Verbindungen der allgemeinen Formel (II), 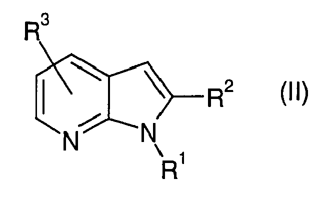 worin R¹, R² und R³ die für die allgemeine Formel (I) genannte Bedeutung haben,
hergestellt werden, wobei die Verbindungen der allgemeinen Formel (II) zunächst mittels Halogenierung in Verbindungen der allgemeinen Formel (VI) überführt werden, 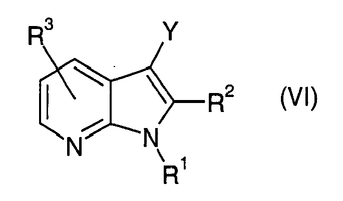 worin Y für Halogen steht, und anschließend mit einem Fluoralkylierungsmittel in Verbindungen der allgemeinen Formel (I) überführt werden, worin X für C₁-C₁₈-Fluoralkyl steht. Prinzipiell sind solche Umsetzungen mit Fluoralkylierungsmittels beispielsweise in M.A. McClinton, Tetrahedron, 1992, 6565-6584 beschrieben.

Weiterhin Gegenstand der vorliegenden Erfindung ist somit ein Verfahren zur Herstellung der erfindungsgemäßen Verbindungen der allgemeinen Formel (I), 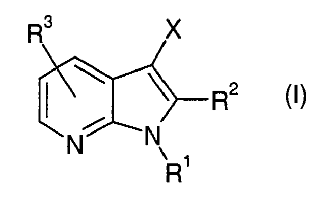 worin X für C₁-C₁₈-Fluoralkyl, bevorzugt C₁-C₆-Fluoralkyl, besonders bevorzugt für CF₃, CF₂CF₃ oder CF(CF₃)₂, ganz besonders bevorzugt für CF₃ steht, und R¹, R² und R³ die vorangehend genannte Bedeutung haben, wobei Verbindungen der allgemeinen Formel (VI), 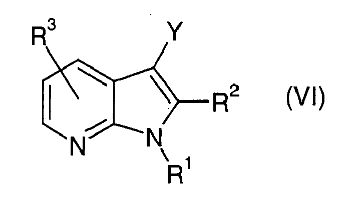 worin Y für Halogen, bevorzugt für I steht, und R¹, R² und R³ die vorangehend genannte Bedeutung haben, mit einem Fluoralkylierungsmittel umgesetzt werden.

Dabei werden die Verbindungen der allgemeinen Formel (VI), worin Y für Halogen, bevorzugt für I steht, und R¹, R² und R³ die vorangehend genannte Bedeutung haben, in einem vorangehenden Schritt beispielsweise aus Verbindungen der allgemeinen Formel (II) mittels Halogenierung hergestellt.

Als Fluoralkylierungsmittel kommen im Rahmen der Erfindung beispielsweise Trialkyl-(perfluoralkyl)-silane der allgemeinen Formel (111),

(R⁴)₃Si(R^{F}) (III)

worin
- R⁴: für gegebenenfalls substituiertes lineares, verzweigtes oder cyclisches C₁-C₁₈-Alkyl, bevorzugt C₁-C₆-Alkyl und
- R^{F}: für lineares, verzweigtes oder cyclisches C₁-C₁₈-Perfluoralkyl, bevorzugt C₁-C₆₋Perfluoralkyl, besonders bevorzugt für CF₃, CF₂CF₃ oder CF(CF₃)₂ steht,

Verbindungen der allgemeinen Formel (IV),

CF₄₋ₙYₙ (IV)

worin
- Y: für Br oder I und
- n: für 1 oder 2 stehen,
oder Trifluoracetate der allgemeinen Formel (V),

CF₃CO₂M (V)

worin
M für Alkalimetallkationen, bevorzugt Na⁺ oder K⁺, oder C₁-C₁₈-Alkyl, bevorzugt C₁-C₆₋Alkyl, besonders bevorzugt Methyl steht.

Bevorzugte Fluoralkylierungsmittel sind Trifluormethylierungsmittel. Bevorzugt sind dies solche der allgemeinen Formel (III), worin R^{F} für CF₃ steht, oder solche der allgemeinen Formel (IV) oder (V).

Das erfindungsgemäße Verfahren wird bevorzugt in Gegenwart von Fluorid-Ionen durchgeführt. Als Fluorid-Ionen-Quelle sind beispielsweise Alkalimetallfluoride oder Tetraalkylammoniumfluoride geeignet. Beispielsweise können dies Kaliumfluorid, Natriumfluorid, Cäsiumfluorid oder Tetraalkylammoniumfluorid sein. Besonders bevorzugt ist Kaliumfluorid. Die Menge des eingesetzten Fluorids kann z.B. das 1- bis 5-fache, bevorzugt, das 1,5- bis 3-fache bezogen auf die Menge an auszutauschendem X (Halogen) in der allgemeinen Formel (I) sein.

Das erfindungsgemäße Verfahren wird bevorzugt in Gegenwart eines Katalysators durchgeführt. Als Katalysatoren sind beispielsweise Zink (für CF₂Br₂ als Fluoralkylierungsmittel), Kupfer (für CF₂Br₂ oder CF₃I oder CF₃Br als Fluoralkylierungsmittel) oder Kupfer(I)-Salze (für Trifluoracetate der allgemeinen Formel (V) und Trialkyl-(perfluoralkyl)-silane der allgemeinen Formel (III) als Fluoralkylierungsmittel) geeignet. Bevorzugt sind Kupfer(I)-Salze, besonders bevorzugt ist Kupfer(I)iodid oder Kupfer(I)cyanid. Die Menge des eingesetzten Katalysators kann z.B. das 0,5-bis 5-fache , bevorzugt das 1,5- bis 3-fache bezogen auf die Menge an auszutauschendem X (Halogen) in der allgemeinen Formel (I) sein.

Die Umsetzung mit dem Fluoralkylierungsmittel wird bevorzugt in Gegenwart von einem oder mehreren Lösungsmittel(n) durchgeführt, bevorzugt in Gegenwart von einem oder mehreren aprotischen Lösungsmittel(n), besonders bevorzugt in Gegenwart von einem oder mehreren dipolar aprotischen Lösungsmittel(n) durchgeführt.

Bevorzugte dipolar aprotische Lösungsmittel sind:

Carbonsäurederivate wie z.B Acetonitril oder N,N-Dimethylformamid (DMF), N,N-Dimethylacetamid (DMAc), N-Methylpyrrolidon (NMP) oder Dimethylimidazolidinon (DMI), Sulfone oder Sulfoxide, wie z.B. Sulfolan oder Dimethylsulfoxid (DMSO) oder Mischungen aus zwei oder mehreren dieser Lösungsmittel. Besonders bevorzugt sind N-Methylpyrrolidon (NMP), N,N-Dimethylformamid (DMF), Dimethylimidazolidinon (DMI), N,N-Dimethylacetamid (DMAc).

Das erfindungsgemäße Verfahren wird bevorzugt bei Temperaturen von -10°C bis 150°C, besonders bevorzugt 0°C bis 100°C, ganz besonders bevorzugt bei 20°C bis 80°C durchgeführt. Die Reaktionszeit beträgt bevorzugt mehrere Stunden, besonders bevorzugt 0,2 bis 24 h, ganz besonders bevorzugt 1 bis 18 h.

Die Durchführung des erfindungsgemäßen Verfahrens unter Schutzgasatmosphäre, wie z.B. Stickstoff- oder Argonatmosphäre, kann von Vorteil sein, ist jedoch nicht zwingend erforderlich.

Das erfindungsgemäße Verfahren kann bei normalem, erhöhtem oder vermindertem Druck durchgeführt werden, beispielsweise im Bereich von 0,5 bis 5 bar. Im Allgemeinen wird es bei Normaldruck durchgeführt.

Das erfindungsgemäße Verfahren wird beispielsweise so durchgeführt, dass die Verbindung der allgemeinen Formel (I), worin X für Halogen steht, in Gegenwart eines Gemisches aus Kaliumfluorid und Cu(I)iodid in dem oder den entsprechenden Lösungsmittel(n) vorlegt und das Fluoralkylierungsmittel zudosiert und das Reaktionsgemisch bei der angegebenen Reaktionstemperatur gerührt wird. Nach beendeter Reaktion wird das Reaktionsgemisch auf wenigstens ein mit dipolar aprotischen Lösungsmitteln nicht oder wenig mischbares organisches Lösungsmittel gegossen und gegebenenfalls mehrmals mit dem oder den mit dipolar aprotischen Lösungsmitteln nicht oder wenig mischbaren organischen Lösungsmittel extrahiert. Nach Entfernen des oder der Lösungsmittel(s) kann die Verbindung der allgemeinen Formel (I) isoliert werden. Zur Erniedrigung der Mischbarkeit kann dem dipolar aprotischen Lösungsmittel Wasser zugesetzt werden.

Als mit dipolar aprotischen Lösungsmitteln gegebenenfalls nach Zugabe von Wasser nicht oder wenig mischbare organische Lösungsmittel kommen beispielsweise Ether, Aliphaten oder Cycloaliphaten in Frage. Beispiele solcher mit dipolar aprotischen Lösungsmitteln nicht oder wenig mischbaren organischen Lösungsmittel sind Methyl-tert.butylether, Hexan, Heptan, Cyclohexan, Methylcyclohexan.

In einer bevorzugten Ausführungsform tragen die Verbindungen der allgemeinen Formel (VI), worin Y für Halogen steht, an der aromatischen NH-Gruppe eine Schutzgruppe, die die oben für R¹ genannten Bedeutungen außer H haben kann, bevorzugt eine -SO₂R^{x} oder -C(O)OR^{x}-Gruppe, worin R^{x} für gegebenenfalls substituiertes C₁-C₁₈-Alkyl, C₄-C₂₄-Aryl oder C₅-C₁₈-Arylalkyl, bevorzugt o-, m- oder insbesondere p-Toluolsulfonyl oder t-Butyloxycarbonyl. Diese Schutzgruppe kann vor oder nach der Umsetzung mit dem Fluoralkylierungsmittel entfernt werden. Es ist auch möglich, die Verbindungen der allgemeinen Formel (I) enthaltend diese Schutzgruppe an der aromatischen NH-Gruppe gezielt herzustellen.

In einer weiteren bevorzugten Ausführungsform erfolgt das Entfernen der Schutzgruppe an der aromatischen NH-Gruppe und die Umsetzung mit dem Fluoralkylierungsmittel in einem Schritt.

Beispielhaft für das vorangehend beschriebene erfindungsgemäße Verfahren können die Verbindungen der allgemeinen Formel (I), worin X für CF₃ steht, aus Verbindungen der allgemeinen Formel (II) zunächst mittels Halogenierung in Verbindungen der allgemeinen Formel (VI), worin Y für Halogen steht, und anschließend durch Umsetzung mit Trialkyl-(perfluoralkyl)-silanen, bevorzugt Trimethyltrifluormethylsilan, oder mit Trifluoracetaten, bevorzugt Natrium- und Kaliumtrifluoracetat oder Methyltrifluoracetat, in Gegenwart von Kupfer(I)salzen, bevorzugt in Gegenwart von Kupfer(I)iodid, und Alkalimetallfluoriden, bevorzugt Kalium- oder Cäsiumfluorid, und in einem dipolar aprotischem Lösungsmittel, wie z.B. N-Methylpyrrolidon (NMP) oder N,N-Dimethylformamid (DMF), oder durch Umsetzung mit Dibromdifluormethan in Gegenwart von Kupfer und in einem dipolar aprotischem Lösungsmittel wie z.B. N,N-Dimethylacetamid (DMAc) hergestellt werden. Über Verbindungen der allgemeinen Formel (VI), worin X für Br steht, lassen sich Verbindungen der allgemeinen Formel (I), worin X für CF₃ steht, auch durch Umsetzung mit Iodtrifluormethan in Gegenwart von Zn oder mit Bromtrifluormethan in Gegenwart von TMEDA (Tetramethylethylendiamin) und Tetraalkylammoniumhalogeniden, z.B. (Bu₄N)Br, herstellen. (vgl. hierzu M. A. McClinton, Tetrahedron, 1992, 6565-6584)

Auch können die erfindungsgemäßen Verbindungen der allgemeinen Formel (I), worin X für CF₃ steht, aus Verbindungen der allgemeinen Formel (VI), worin Y für COR⁵ steht, wobei R⁵ für Halogen, OH oder gegebenenfalls substituiertes lineares, verzweigtes oder cyclisches C₁-C₁₈₋Alkyloxy, bevorzugt C₁-C₆-Alkyloxy, C₄-C₂₄-Aryloxy, bevorzugt C₆-C₂₄-Aryloxy, oder C₅-C₁₈₋Arylalkyloxy steht, durch Umsetzung mit SF₄/HF hergestellt werden. Solche Reaktionen mit SF₄/HF oder DAST/NaF sind beispielsweise in Wang C.-LJ., Org. React. 34, 319-400 oder Hudlicky, M., Org. React. 35, 513-637 beschrieben.

Weiterhin Gegenstand der vorliegenden Erfindung ist somit ein Verfahren zur Herstellung der erfindungsgemäßen Verbindungen der allgemeinen Formel (I), 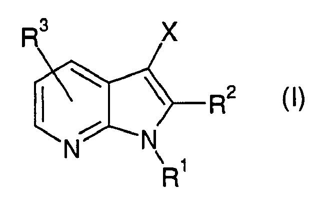 worin X, R¹, R² und R³ die vorangehend genannte Bedeutung haben, wobei Verbindungen der allgemeinen Formel (VI), 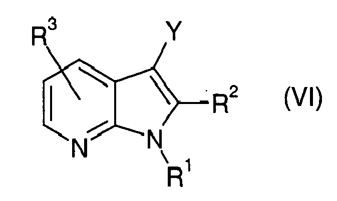 worin Y für COR⁵ steht, wobei R⁵ für Halogen, OH oder gegebenenfalls substituiertes lineares, verzweigtes oder cyclisches C₁-C₁₈-Alkyloxy, bevorzugt C₁-C₆-Alkyloxy, C₄-C₂₄-Aryloxy, bevorzugt C₆-C₂₄-Aryloxy, oder C₅-C₁₈-Arylalkyloxy steht, und R¹, R² und R³ die vorangehend genannte Bedeutung haben, mit SF₄ gegebenenfalls in Gegenwart von HF oder mit Diethylaminoschwefeltrifluorid (DAST) gegebenenfalls in Gegenwart von NaF umgesetzt werden.

Die Herstellung der Verbindungen der allgemeinen Formel (VI), worin Y für COR⁵ steht, kann beispielsweise wie in J. Amer. Chem. Soc. 1956, 78, 1247-1249 beschrieben erfolgen.

Das erfindungsgemäße Verfahren wird bevorzugt in Gegenwart eines Überschusses HF durchgeführt. Die Menge des HF kann z.B. 10 bis 30 Äquivalente bezogen auf die Menge der Verbindung der allgemeinen Formel (VI) betragen.

Das erfindungsgemäße Verfahren kann in Gegenwart oder in Abwesenheit von einem oder mehreren Lösungsmittel(n) durchgeführt. Als Lösungsmittel sind beispielsweise halogenierte Kohlenwasserstoffe, wie z.B. Dichlormethan geeignet.

Das erfindungsgemäße Verfahren kann bei normalem, erhöhtem oder vermindertem Druck durchgeführt werden, beispielsweise im Bereich von 0,5 bis 5 bar. Im Allgemeinen wird es bei Normaldruck durchgeführt.

Das erfindungsgemäße Verfahren wird bevorzugt bei Temperaturen von 80°C bis 140°C durchgeführt. Die Reaktionszeit beträgt bevorzugt mehrere Stunden, besonders bevorzugt 0,2 bis 24 h, ganz besonders bevorzugt 1 bis 18 h.

Die Durchführung des erfindungsgemäßen Verfahrens unter Schutzgasatmosphäre, wie z.B. Stickstoff- oder Argonatmosphäre, kann von Vorteil sein, ist jedoch nicht zwingend erforderlich.

Das erfindungsgemäße Verfahren ermöglicht die Herstellung der Verbindungen der allgemeinen Formel (I) unter besonders schonenden Bedingungen. Dadurch können Nebenreaktionen wie beispielsweise Dimerisierungen von Verbindungen der allgemeinen Formel (VI), worin Y für I steht, weitgehend unterbunden werden.

Die Verbindungen der allgemeinen Formel (VI), 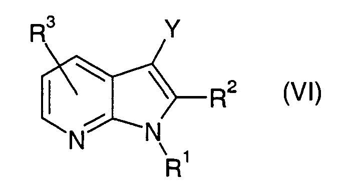 worin
- R¹: für H oder gegebenenfalls substituiertes lineares, verzweigtes oder cylisches C₁-C₁₈-Alkyl, bevorzugt C₁-C₆-Alkyl sowie C₃-C₆-Cycloalkyl, C₁-C₁₈-Alkoxy, bevorzugt C₁-C₆-Alkoxy, C₂-C₆-Alkenyl, C₄-C₆-Cycloalkenyl, C₂-C₆-Alkinyl, C₄-C₂₄-Aryl, bevorzugt C₆-C₂₄-Aryl, C₅-C₁₈-Arylalkyl, C₅-C₁₈-Aryloxy, C₅-C₁₈-Arylalkoxy, -SO₂R^{x}, -SO₂NR^{x}R^{y}, -C(O)OR^{x} oder-C(O)NR^{x}R^{y}, Tri-C₁-C₁₈-alkylsilyl-Gruppen sowie weitere geeignete NH-Schutzgruppen für aromatische Verbindungen,
worin R^{x} und R^{y} unabhängig voneinander für H oder gegebenenfalls substituiertes lineares, verzweigtes oder cylisches C₁-C₁₈-Alkyl, bevorzugt C₁-C₆-Alkyl sowie C₃-C₆-Cycloalkyl, , C₁-C₁₈-Alkoxy, bevorzugt C₁-C₆-Alkoxy, C₂-C₆-Alkenyl, C₄-C₆-Cycloalkenyl, C₂-C₆₋Alkinyl, C₄-C₂₄-Aryl, bevorzugt C₆-C₂₄-Aryl, C₅-C₁₈-Arylalkyl, C₅-C₁₈-Aryloxy oder C₅₋C₁₈-Arylalkoxy steht,
- R² R³: unabhängig voneinander für H, Cl, F, Br, NO₂ Pseudohalogen, Formyl oder geschütztes Formyl, Carboxyl, C(S)NH₂, C(O)NH₂, gegebenenfalls substituiertes lineares, verzweigtes oder cylisches C₁-C₁₈-Alkyl, bevorzugt C₁-C₆-Alkyl sowie C₃-C₆-Cycloalkyl, C₁-C₁₈₋Fluoralkyl, bevorzugt C₁-C₆-Fluoralkyl, C₁-C₁₈-Alkoxy, bevorzugt C₁-C₆-Alkoxy, C₁-C₁₈₋Fluoralkoxy, bevorzugt C₁-C₆-Fluoralkoxy, C₂-C₆-Alkenyl, C₄-C₆-Cycloalkenyl, C₂-C₆₋Alkinyl, C₄-C₂₄-Aryl, bevorzugt C₆-C₂₄-Aryl, C₅-C₁₈-Arylalkyl oder C₅-C₁₈-Arylalkoxy steht, wobei für den Fall, dass R³ für C₅-C₁₈-Arylalkyl steht und dieser über den Alkylteil des C₅₋C₁₈-Arylalkylrestes an den Pyridinring gebunden ist, dieser Alkylteil wenigstens zwei Kohlenstoffatome zwischen Arylteil des C₅-C₁₈-Arylalkylrestes und Pyridinring aufweist, und
- Y: für Halogen oder COR⁵ steht, worin R⁵ für Halogen, OH oder gegebenenfalls substituiertes lineares, verzweigtes oder cylisches C₁-C₁₈-Alkyloxy, bevorzugt C₁-C₆-Alkyloxy, C₄-C₂₄₋Aryloxy, bevorzugt C₆-C₂₄-Aryloxy oder C₅-C₁₈-Arylalkyloxy steht,
wobei wenigstens einer, bevorzugt wenigstens zwei der Reste R¹, R² oder R³ nicht für H stehen,
sind bisher in der Literatur nicht beschrieben und daher ebenfalls Gegenstand der vorliegenden Erfindung.

In einer bevorzugten Ausführungsform steht R¹ in den erfindungsgemäßen Verbindungen der allgemeinen Formel (VI) nicht für H. Besonders bevorzugt steht R¹ in diesen erfindungsgemäßen Verbindungen für -SO₂R^{x}, -SO₂NR^{x}R^{y}, -C(O)OR^{x} oder -C(O)NR^{x}R^{y}, worin R^{x} und R^{y} unabhängig voneinander für H oder gegebenenfalls substituiertes C₁-C₁₈-Alkyl, C₄-C₂₄-Aryl oder C₅-C₁₈₋Arylalkyl stehen, bevorzugt für -SO₂R^{x} oder -C(O)OR^{x}, besonders bevorzugt für o-, m- oder insbesondere p-Toluolsulfonyl oder t-Butyloxycarbonyl.

Beispiele für erfindungsgemäße Verbindungen der allgemeinen Formel (VI) sind beispielsweise die folgenden Verbindungen der Formeln (VI-1) und (VI-2):

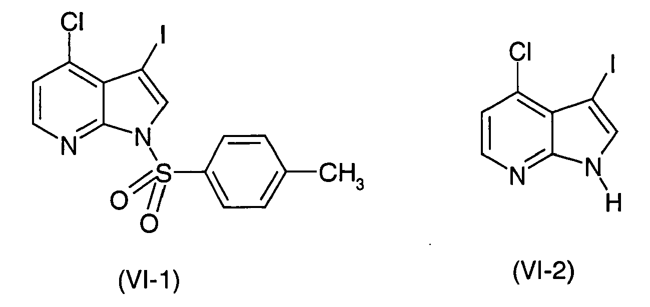

Die erfindungsgemäßen Verbindungen der allgemeinen Formel (VI) eignen sich hervorragend zur Herstellung der Verbindungen der allgemeinen Formel (I), insbesondere mit den vorangehend beschriebenen erfindungsgemäßen Verfahren.

Die erfindungsgemäßen Verbindungen der allgemeinen Formel (I) eignen sich beispielsweise als Zwischenprodukte für die Herstellung pharmazeutischer Wirkstoffe oder weisen selbst pharmazeutische Wirksamkeit auf und eignen sich als pharmazeutische Wirkstoffe. Dabei können die aus den erfindungsgemäßen Verbindungen der allgemeinen Formel (I) hergestellten pharmazeutischen Wirkstoffe oder die erfindungsgemäßen Verbindungen der allgemeinen Formel (I) selbst den bekannten Verbindungen beispielsweise hinsichtlich Löslichkeit, Wirkhöhe oder Pharmakokinetik überlegen sein.

Die folgenden Beispiele dienen der beispielhaften Veranschaulichung der Erfindung und sind nicht als Beschränkung aufzufassen.

### Beispiele

### 1.) Darstellung von 4-Chlor-3-iod-1H-pyrrolo[2,3-b]pyridin (VI-2)

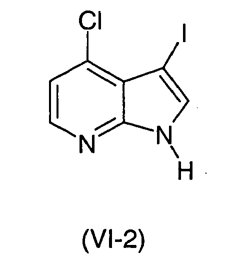

Es wurden 250 g (1.64 mol) 4-Chlor-1*H*-pyrrolo[2,3-b]pyridin zusammen mit 328 g (5.85 mol) pulverförmigen KOH in 3000 ml DMF vorgelegt und auf 0°C abgekühlt. Zu dieser Suspension wurde man unter Kühlung eine Lösung aus 416 g (1.64 mol) Iod in 3000 ml DMF getropft und nach vollständiger Zugabe weitere 4 h bei 0°C gerührt. Nach Vervollständigung der Reaktion wurde der Ansatz unter Rühren auf Eiswasser gegeben, der gebildete Feststoff abgesaugt und anschließend im Hochvakuum getrocknet. Es wurden insgesamt 403 g (1.45 mol, 88%) 4-Chlor-3-iod-1*H*-pyrrolo[2,3-b]pyridin als farbloser Feststoff isoliert.
1H-NMR (DMSO): 7.21 (d, 1H, H-arom), 7.81 (d, 1H, H-arom), 8.19 (d, 1H, H-arom), 12.45 (br s, 1H, N-H).
LC-MS: 278 [M+]

### 2.) Darstellung von 4-Chlor-1-(toluol-4-sulfonyl)-3-iod-1H-pyrrolo[2,3-b]pyridin (VI-1)

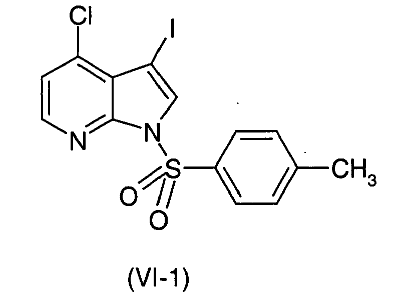

Unter Schutzgasatmosphäre (Argon) wurden 63.5 g (1.59 mol) Natriumhydrid in 1000 ml abs. THF suspendiert. Hierzu tropfte man unter Kühlung bei einer Temperatur von 0-5°C eine Lösung aus 402 g (1.44 mol) 4-Chlor-3-iod-1*H*-pyrrolo[2,3-b]pyridin in 3000 ml abs. THF und ließ nach vollständiger Zugabe das Reaktionsgemisch 15 Minuten nachrühren. Anschließend wurden 358 g (1.88 mol) p-Toluolsulfonsäurechlorid portionsweise zum Reaktionsgemisch zugegeben, so dass die Temperatur zwischen 5-10°C gehalten wurde. Nach Beendigung der Zugabe ließ man den Ansatz auf 20°C kommen und rührte eine weitere Stunde bei dieser Temperatur. Nach vollständigem Umsatz (DC-Umsatzkontrolle) wurde das Reaktionsgemisch mit 20 1 gesättigter NaHCO₃-Lösung sowie 10 1 Ethylacetat versetzt, extrahiert und die organische Phase abgetrennt. Die wässrige Phase wurde noch zweimal mit je 10 1 Ethylacetat nachextrahiert, die organischen Phasen vereinigt, getrocknet und im Wasserstrahlvakuum von flüchtigen Bestandteilen befreit. Zur Aufreinigung des kristallinen Rohproduktes wurde dieses in Dichlormethan aufgenommen und über Kieselgel chromatographiert (CH₂Cl₂ : Petrolether 40/60 = 3 : 7). Nach Abziehen der Lösungsmittel wurde der Kristallbrei in Diethylether ausgerührt, abgesaugt und im Hochvakuum bei 20°C getrocknet. Es wurden insgesamt 372 g (0.86 mol, 60%) 4-Chlor-1-(toluol-4-sulfonyl)-3-iod-1*H*-pyrrolo[2,3-b]pyridin als farbloser Feststoff isoliert.
1H-NMR (DMSO): 2.35 (s, 3H, -CH₃), 7.44 (d, 2H, H-arom), 7.46 (d, 1H, H-arom), 8.02 (d, 2H, H-arom), 8.23 (s, 1H, H-arom), 8.34 (d, 1H, H-arom).
LC-MS: 432 [M+]

### 3.) Darstellung von 4-Chlor-1-(toluol-4-sulfonyl)-3-trifluormethyl-1H-pyrrolo[2,3-b]pyridin (I-4)

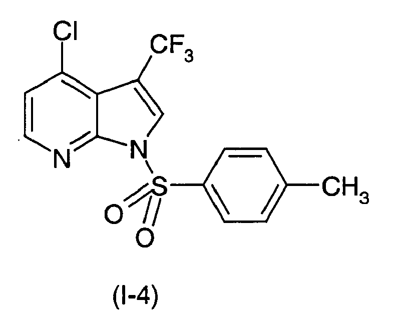

Unter Schutzgasatmosphäre (Argon) wurden 23.1 g (0.398 mol) trockenes Kaliumfluorid und 75.7 g (0.398 mol) Kupfer(I)iodid miteinander verrieben und im Hochvakuum unter Schütteln 10 Minuten auf 220°C erhitzt, bis eine leicht grünliche Verfärbung entstand. In einem Reaktionskolben wurden unter Schutzgasatmosphäre (Argon) zunächst das geglühte Gemisch aus Kaliumfluorid und Kupfer(I)iodid zusammen mit 170 ml absolutiertem NMP und 170 ml absolutiertem DMF vorgelegt und anschließend bei 20°C unter Rühren 86 g (0.199 mol) 4-Chlor-1-(toluol-4-sulfonyl)-3-iod-1*H*-pyrrolo[2,3-b]pyridin portionsweise eingetragen, wobei sich eine hellgraue Suspension bildet. Man tropfte innerhalb von 20 Minuten 62.1 g (0.437 mol) Trimethyltrifluormethylsilan zum Reaktionsgemisch hinzu und ließ 18 h bei 20°C rühren. Nach vollständigem Umsatz (GC-Umsatzkontrolle) wurde das Reaktionsgemisch auf 2000 ml Methyl-tert.butylether gegeben, die organische Phase dekantiert und der Rückstand ein weiteres Mal mit 1000 ml Methyl-tert.butylether dispergiert. Die vereinten organischen Phasen wurden dreimal mit je 2000 ml Wasser gewaschen und anschließend über MgSO₄ getrocknet. Nach Filtration und Entfernen der flüchtigen Bestandteile im Wasserstrahlvakuum resultieren 50.1 g (0.134 mol; 67 %) 4-Chlor-1-(toluol-4-sulfonyl)-3-trifluormethyl-1*H*-pyrrolo[2,3-b] pyridin als beiger Feststoff.
1H-NMR (DMSO): 2.37 (s, 3H, -CH₃), 7.48 (d, 2H, H-arom), 7.62 (d, 1H, H-arom), 8.12 (d, 2H, H-arom), 8.47 (d, 1H, H-arom), 8.67 (s, 1H, H-arom).
LC-MS: 374 [M+]

### 4.) Darstellung von 4-Chlor-3-trifluormethyl-1H-pyrrolo[2,3-b]pyridin (I-2)

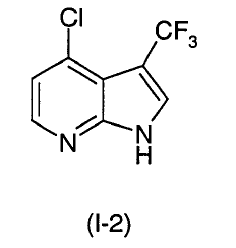

Zu einer Lösung von 30 g (80.0 mmol) 4-Chlor-1-(toluol-4-sulfonyl)-3-trifluormethyl-1*H-*pyrrolo[2,3-b]pyridin in 250 ml absolutiertem THF gibt man 52.3 g (200 mmol) Tetra-n-butylammoniumfluorid und rührt bei 20°C für 20 Minuten. Nach vollständigem Umsatz (DC-Kontrolle) versetzt man mit 300 ml gesättigter NaHCO₃-Lösung und extrahiert anschliessend dreimal mit je 500 ml Ethylacetat. Die vereinten organischen Phasen werden mit gesättigter NaCl-Lösung gewaschen, über MgSO₄ getrocknet und im Wasserstrahlvakuum von flüchtigen Bestandteilen befreit. Nach Aufreinigung des kristallinen Rohproduktes durch Chromatographie über Kieselgel (CH₂Cl₂ : MeOH = 30 : 1) resultieren 13.1 g (59.5 mmol; 74%) 4-Chlor-3-trifluormethyl-1*H*-pyrrolo[2,3-b]pyridin als beiger Feststoff.
1H-NMR (DMSO): 7.40 (d, 1H, H-arom), 8.28 (s, 1H, H-arom), 8.34 (d, 1H, H-arom), 12.88 (br s 1H, N-H).
LC-MS: 220 [M+]

## Patentansprüche

1. Verbindung der allgemeinen Formel (I), 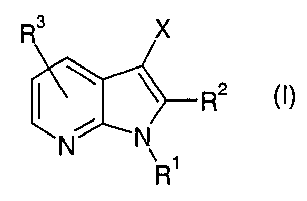 worin
R¹ für H oder gegebenenfalls substituiertes lineares, verzweigtes oder cyclisches C₁₋C₁₈-Alkyl, C₁-C₁₈-Alkoxy, C₂-C₆-Alkenyl, C₄-C₆-Cycloalkenyl, C₂-C₆-Alkinyl, C₄-C₂₄-Aryl, C₅-C₁₈-Arylalkyl, C₅-C₁₈-Aryloxy, C₅-C₁₈-Arylalkoxy, -SO₂R^{x}, -SO₂NR^{x}R^{y}, -C(O)OR^{x}, -C(O)NR^{x}R^{y} oder Tri-C₁-C₁₈-alkylsilyl-Gruppen,
worin R^{x} und R^{y} unabhängig voneinander für H oder gegebenenfalls substituiertes lineares, verzweigtes oder cyclisches C₁-C₁₈-Alkyl, C₁-C₁₈-Alkoxy, C₂-C₆₋Alkenyl, C₄-C₆-Cycloalkenyl, C₂-C₆-Alkinyl, C₄-C₂₄-Aryl, C₅-C₁₈-Arylalkyl, C₅₋C₁₈-Aryloxy oder C₅-C₁₈-Arylalkoxy steht,
R², R³ unabhängig voneinander für H, Cl, F, Br, NO₂, Pseudohalogen, Formyl oder geschütztes Formyl, Carboxyl, C(S)NH₂, C(O)NH₂, gegebenenfalls substituiertes lineares, verzweigtes oder cyclisches C₁-C₁₈-Alkyl, C₁-C₁₈-Fluoralkyl, C₁-C₁₈₋Alkoxy, C₁-C₁₈-Fluoralkoxy, C₂-C₆-Alkenyl, C₄-C₆-Cycloalkenyl, C₂-C₆-Alkinyl, C₄-C₂₄-Aryl, C₅-C₁₈-Arylalkyl oder C₅-C₁₈-Arylalkoxy steht, wobei für den Fall, dass R³ für C₅-C₁₈-Arylalkyl steht und dieser über den Alkylteil des C₅-C₁₈₋Arylalkylrestes an den Pyridinring gebunden ist, dieser Alkylteil wenigstens zwei Kohlenstoffatome zwischen Arylteil des C₅-C₁₈-Arylalkylrestes und Pyridinring aufweist, und
X für C₁-C₁₈-Fluoralkyl steht,
sowie Salze der Verbindungen der allgemeinen Formel (I).

2. Verbindung nach Anspruch 1, **dadurch gekennzeichnet, dass** R¹ für H oder -SO₂R^{x} oder - C(O)OR^{x}, worin R^{x} für gegebenenfalls substituiertes C₁-C₁₈-Alkyl, C₄-C₂₄-Aryl oder C₅-C₁₈₋Arylalkyl steht, bevorzugt für H, p-Toluolsulfonyl oder t-Butyloxycarbonyl steht.

3. Verbindungen nach Anspruch 1 oder 2, **dadurch gekennzeichnet, dass** R² für H oder C₁-C₆₋Alkyl, C₆-C₂₄-Aryl oder C₅-C₁₈-Arylalkyl steht.

4. Verbindungen nach wenigstens einem der Ansprüche 1 bis 3, **dadurch gekennzeichnet, dass** R³ für H, Cl, F, Br, NO₂, CN, Formyl oder geschütztes Formyl, C₁-C₆-Alkyl, C₁-C₆₋Fluoralkyl, C₁-C₆-Alkoxy, C₁-C₆-Fluoralkoxy, C₆-C₂₄-Aryl, C₅-C₁₈-Arylalkyl, C₅-C₁₈₋Arylalkoxy, bevorzugt für H oder Cl steht.

5. Verbindungen nach wenigstens einem der Ansprüche 1 bis 4, **dadurch gekennzeichnet, dass** X für C₁-C₆-Fluoralkyl, bevorzugt für CF₃, CF₂CF₃ oder CF(CF₃)₂ steht.

6. Mischung enthaltend eine oder mehrere Verbindung(en) nach wenigstens einem der Ansprüche 1 bis 5.

7. Verfahren zur Herstellung von Verbindungen der allgemeinen Formel (I), 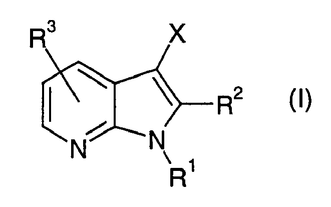 worin X, R¹, R² und R³ die in wenigstens einem der Ansprüche 1 bis 4 genannte Bedeutung haben, **dadurch gekennzeichnet, dass** Verbindungen der allgemeinen Formel (VI), 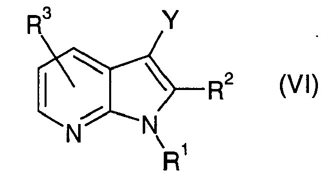 worin Y für Halogen, bevorzugt für I steht, und R¹, R² und R³ die in wenigstens einem der Ansprüche 1 bis 4 genannte Bedeutung haben mit einem Fluoralkylierungsmittel umgesetzt werden.

8. Verfahren nach Anspruch 7, **dadurch gekennzeichnet, dass** X für CF₃ steht und das Fluoralkylierungsmittel ein Trifluormethylierungsmittel ist.

9. Verfahren nach Anspruch 7 oder 8, **dadurch gekennzeichnet, dass** die Umsetzung in Gegenwart von einem oder mehreren Lösungsmittel(n) durchgeführt wird.

10. Verfahren nach wenigstens einem der Ansprüche 7 bis 9, **dadurch gekennzeichnet, dass** die Herstellung der Verbindungen der allgemeinen Formel (I), worin R¹ für H steht und X, R² und R³ die in wenigstens einem der Ansprüche 1 bis 4 genannte Bedeutung haben, in Gegenwart von Fluorid-Ionen erfolgt.

11. Verfahren nach wenigstens einem der Ansprüche 7 bis 10, **dadurch gekennzeichnet, dass** die Herstellung der Verbindungen der allgemeinen Formel (1), worin R¹ für H steht und X, R² und R³ die in wenigstens einem der Ansprüche 1 bis 4 genannte Bedeutung haben, in Gegenwart eines Katalysators erfolgt.

12. Verfahren nach wenigstens einem der Ansprüche 7 bis 11, **dadurch gekennzeichnet, dass** die Verbindung der allgemeinen Formel (I), worin R¹ für H steht und X, R² und R³ die in wenigstens einem der Ansprüche 1 bis 4 genannte Bedeutung haben, nach der Umsetzung mit dem Fluoralkylierungsmittel aus Verbindungen der allgemeinen Formel (I), worin R¹ für-SO₂R^{x} oder -C(O)OR^{x}, worin R^{x} für gegebenenfalls substituiertes C₁-C₁₈-Alkyl, C₄-C₂₄-Aryl oder C₅-C₁₈-Arylalkyl steht, bevorzugt für H, p-Toluolsulfonyl oder t-Butyloxycarbonyl steht, und X, R² und R³ die in wenigstens einem der Ansprüche 1 bis 4 genannte Bedeutung haben, hergestellt werden.

13. Verfahren zur Herstellung von Verbindungen der allgemeinen Formel (I), 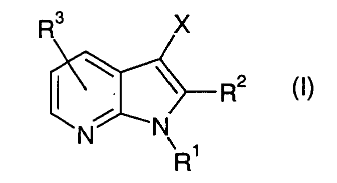 worin X, R¹, R² und R³ die in wenigstens einem der Ansprüche 1 bis 4 genannte Bedeutung haben, **dadurch gekennzeichnet, dass** Verbindungen der allgemeinen Formel (VI), 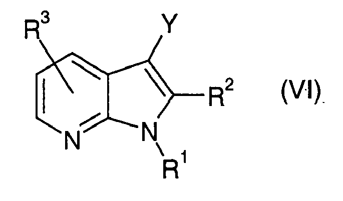 worin
Y für COR⁵ steht, worin R⁵ für Halogen, OH oder gegebenenfalls substituiertes lineares, verzweigtes oder cyclisches C₁-C₁₈-Alkyloxy, bevorzugt C₁-C₆-Alkyloxy, C₄-C₂₄-Aryloxy, bevorzugt C₆-C₂₄-Aryloxy oder C₅-C₁₈-Arylalkyloxy steht, und R¹, R² und R³ die in wenigstens einem der Ansprüche 1 bis 4 genannte Bedeutung haben, mit SF₄ gegebenenfalls in Gegenwart von HF umgesetzt werden.

14. Verbindung der allgemeinen Formel (VI), 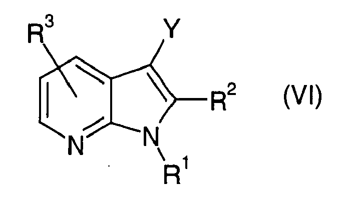 worin
R¹ für H oder gegebenenfalls substituiertes lineares, verzweigtes oder cyclisches C₁₋C₁₈-Alkyl, C₁-C₁₈-Alkoxy, C₂-C₆-Alkenyl, C₄-C₆-Cycloalkenyl, C₂-C₆-Alkinyl, C₄-C₂₄-Aryl, C₅-C₁₈-Arylalkyl, C₅-C₁₈-Aryloxy, C₅-C₁₈-Arylalkoxy, -SO₂R^{x}, -SO₂NR^{x}R^{y}, -C(O)OR^{x}, -C(O)NR^{x}R^{y} oder Tri-C₁-C₁₈-alkylsilyl-Gruppen,
worin R^{x} und R^{y} unabhängig voneinander für H oder gegebenenfalls substituiertes lineares, verzweigtes oder cyclisches C₁-C₁₈-Alkyl, C₁-C₁₈-Alkoxy, C₂-C₆₋Alkenyl, C₄-C₆-Cycloalkenyl, C₂-C₆-Alkinyl, C₄-C₂₄-Aryl, C₅-C₁₈-Arylalkyl, C₅₋C₁₈-Aryloxy oder C₅-C₁₈-Arylalkoxy steht,
R², R³ unabhängig voneinander für H, Cl, F, Br, NO₂, Pseudohalogen, Formyl oder geschütztes Formyl, Carboxyl, C(S)NH₂, C(O)NH₂, gegebenenfalls substituiertes lineares, verzweigtes oder cyclisches C₁-C₁₈-Alkyl, C₁-C₁₈-Fluoralkyl, C₁-C₁₈₋Alkoxy, C₁-C₁₈-Fluoralkoxy, C₂-C₆-Alkenyl, C₄-C₆-Cycloalkenyl, C₂-C₆-Alkinyl, C₄-C₂₄-Aryl, C₅-C₁₈-Arylalkyl oder C₅-C₁₈-Arylalkoxy steht, wobei für den Fall, dass R³ für C₅-C₁₈-Arylalkyl steht und dieser über den Alkylteil des C₅-C₁₈₋Arylalkylrestes an den Pyridinring gebunden ist, dieser Alkylteil wenigstens zwei Kohlenstoffatome zwischen Arylteil des C₅-C₁₈-Arylalkylrestes und Pyridinring aufweist, und
Y für Halogen oder COR⁵ steht, worin R⁵ für Halogen, OH oder gegebenenfalls substituiertes lineares, verzweigtes oder cylisches C₁-C₁₈-Alkyloxy, C₄-C₂₄₋Aryloxy, oder C₅-C₁₈-Arylalkyloxy steht,
wobei wenigstens einer der Reste R¹, R² oder R³ nicht für H steht.

15. Verwendung der Verbindungen der allgemeinen Formel (VI) zur Herstellung von Verbindungen der allgemeinen Formel (I).

16. Verwendung der Verbindungen der allgemeinen Formel (I) als Zwischenprodukte für die Herstellung pharmazeutischer Wirkstoffe.
